# EUROPEAN PATENT SPECIFICATION

(11) **EP 2 111 168 B1**
(45) Date of publication and mention of the grant of the patent: **20.03.2013**
(21) Application number: 07866570.0
(22) Date of filing: 20.12.2007
(51) Int. Cl.: A61B 17/16

(54) **DISPOSABLE ACETABULAR REAMER FROM FLAT STOCK**
WEGWERFBARER PFANNENFRÄSER AUS EINEM FLACHEN MATERIAL
ALÉSOIR COTYLOÏDIEN JETABLE FORMÉ À PARTIR D'UNE DÉCOUPE PLATE

(30) Priority: 23.12.2006 US 871780 P
(43) Date of publication of application: 28.10.2009
(73) Proprietor: Greatbatch Medical SA, 2534 Orvin (CH)
(72) Inventor: LECHOT, André, 2534 Orvin (CH); CLAUDE, Sarah, 25500 La Chenalotte (FR); FEHLBAUM, Philippe, 2523 Lignieres (CH); WHITE, Patrick, West Chester, PA 19382 (US); GUELAT, Didier, 2900 Porrentruy (CH)
(74) Representative: Koepe & Partner
(86) International application number: PCT/IB2007/004030
(87) International publication number: WO 2008/078158

(56) References cited:
- EP-A- 0 879 577
- WO-A-99/47051
- WO-A-2004/100804
- WO-A-2007/097749
- FR-A- 2 281 095
- US-A- 3 605 527
- US-A- 4 811 632
- US-A- 5 100 267
- US-A1- 2005 113 837

## Description

### Cross reference to Related Applications:

This application claims priority to US provisional application serial no. 60/871,780 of the same name, filed December 23, 2006.

### Background of the Invention

This invention relates to tools for cutting bone, in particular, to reamers for cutting the cotyloid cavity of the acetabulum in the event of the replacement of the hip joint by a prosthetic cup.

Document US 5,100,267 discloses a disposable acetabular reamer cup which has a cutting bowl having a plurality of cutting edges. The cutting bowl has perforations adjoining the cutting edges. The cutting bowl defines an axis of rotation. The cutting bowl has a bottom opening. A polymeric plug is joined to the cutting bowl. The plug is concentric with the axis of rotation. The plug occupies the bottom opening. The plug has a tool driver opening concentric with the axis of rotation.

Tools for cutting bone are often difficult to clean, and prions, soft tissue embedded in the tool from some prior use, are often difficult to remove. It is considered the most certain manner in which to ensure that no cross contamination occurs between uses is to provide a cutting tool that is intended for a single use. After such use, the cutting tool is simply discarded.

Acetabular reamers in the prior art are generally press-formed out of stamped, flat metal stock. However, in the typical such case, the hemispherical form that results after press-forming can include deformaties, considering the severe working and stresses induced in the materials as the flat stock is formed up into a hemispherical shape. To obtain an accurate result with minimum warpage, such reamers must be made with complex and expensive machinery. Consequently, the costs for such prior art reamers are high. High costs of manufacture mean high final cost. Customers which pay a significant amount for a tool are not likely to discard such tools after a single use.

Nothing herein is to be construed as an admission that the present invention is not entitled to antedate such cited material by virtue of prior invention.

What is needed therefore is a tool for cutting bone that is simple to manufacture at low cost and therefore disposable.

Still further, what is needed therefore is a method of manufacturing a disposable reamer that provides an accurate hemispherical form and thus ensures accurate cutting.

### Summary of the Invention

The invention relates to a reamer assembly according to claim 1.

An at least partially disposable reamer is provided, made up of a circular base to which a press-formed, thin-walled, perforated cutting body of flat metal stock may be mechanically affixed. Once affixed, the assembly is rigid enough for cutting bone, yet may be disassembled so as to permit partial disposal. Prior to press-forming, the flat metal body is stamped out of flat metal stock, optionally having a plurality of appendages connected at a center. The base and body may be molded together or assembled together in a mechanical manner.

An object of the invention is that the appendages assure improved die forming of the body into a final desired shape while at the same time minimizing stress-induced deformation in the body.

### Brief Description of the Drawings

FIG. **1A** is a perspective view of the disposable reamer of the invention.
FIG. **1B** is a perspective view of an alternate emdbodiment of the disposable reamer of the invention.
FIG. **2** is a perspective view showing the base of the reamer assembly of the invention.
FIG. **3A** is a perspective view of a blank from which the cutting body of the reamer assembly of the invention is made.
FIG. **3B** is a perspective view of an alternate blank from which the cutting body of the reamer assembly of the invention is made.
FIG. **3C** is a perspective view of an alternate blank from which the cutting body of the reamer assembly of the invention is made.
FIG. **3D** is a perspective view of an alternate blank from which the cutting body of the reamer assembly of the invention is made.
FIG. **3E** is a perspective view of an alternate blank from which the cutting body of the reamer assembly of the invention is made.
FIG. **4A** is a perspective view of a further alternate blank from which the cutting body of the reamer assembly of the invention is made.
FIG. **4B** is a perspective view of a further alternate blank from which the cutting body of the reamer assembly of the invention is made.
FIG. **5** is a side, partial break-away view of the reamer assembly of the invention.
FIG. **6** is a flow chart of the method of manufacturing the reamer assembly of the invention.
FIG. 7 is a perspective, exploded view showing the pre-assembled components of the reamer of the invention.

### Detailed Description of the Preferred embodiment(s)

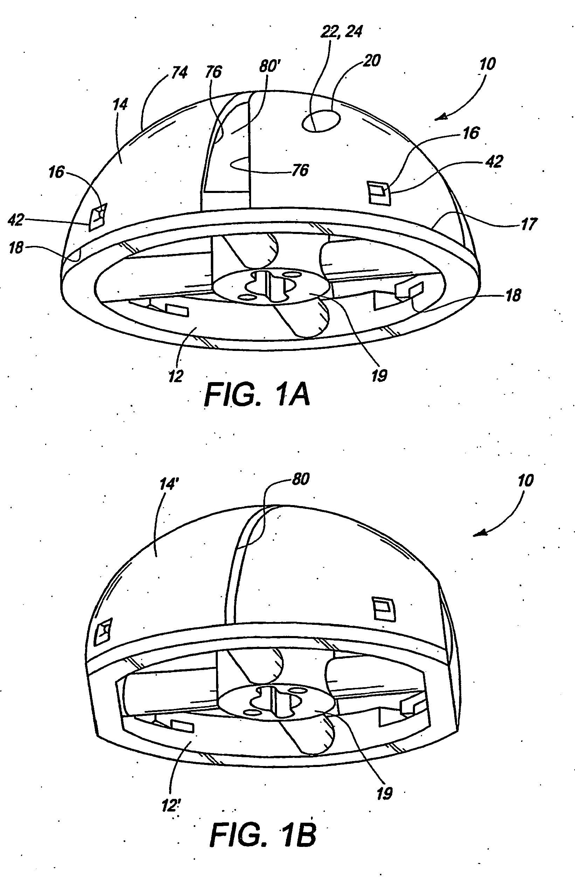

Referring now to FIGs. **1A**, a reamer assembly 10 is made up of a base 12 and a cutting body 14 having locking interfaces 16 which lock with the base so as to permit interlocking therebetween. Locking interface 18 may be used for mechanical locking of the base 12 and cutting body 14 together.

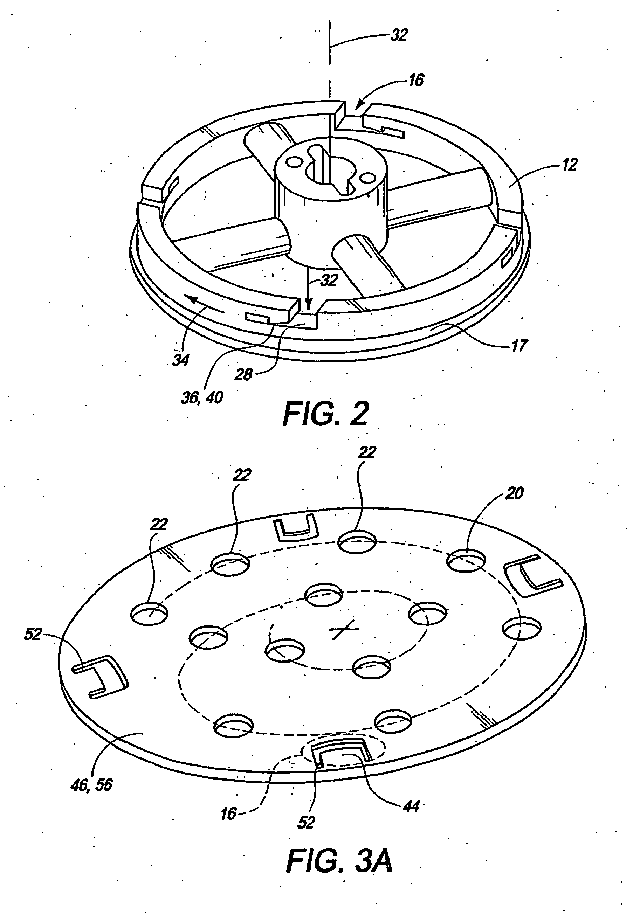

Referring to FIG. **2**, the base 12 is rigid, preferably made of a biocompatible plastic, and has a form which is circular, preferably including a stabilizing flange 17, against which the equatorial edge 18 of the cutting body 14 may abut. The base 12 includes an interface 19 (FIGS. 1A and 1B) for interfacing with a holder (not shown), such as that described in US Provisional Application No. 60/971406, filed 21 December, 2006, connected to a rotating power source (not shown).

The cutting body 14 is a die-formed or press-formed, thin walled, convex member having perforations 20 thereon formed at cutting sites 22. Cutting teeth 24 are formed adjacent the perforations 20, which are distributed on the cutting body 14 so as to evenly cut bone when rotated with a cutting tool (not shown) in the acetabulum, in order to prepare the same for reception of a prosthesis (not shown)

Referring now to FIG. **1B**, an alternate embodiment is shown, in which a reamer assembly 10' has a base 12' which is not circular, but rather formed to minimize it's insertion profile during minimally invasive surgery, such as described in US patent application ser. No. 10/510,934 filed 08/01/2002. In this embodiment, a cutting body 14' has a corresponding form.

Referring now to FIG. 2, the base 12, 12' and the cutting body 14, 14' interlock via, for example, a snap-type, bayonet interface 16 comprised of a bayonet slot 28 which runs in a direction parallel to the axis 32 of rotation of the reamer assembly 10, when rotated for cutting, then doglegs in a circumferential direction 34, at which point is found a locking device 36 such as a spur 40 which ensures that the connection 42 therebetween is of a rigidity sufficient to permit cutting while also, where desired, separation of the base 12 from the cutting body 14.

Referring now to FIG. **3A**, in one embodiment, the locking interface 16 of the cutting body 14 is a simple tab 44 which, after perforation, is die-formed at 90 degrees to the plane of the blank 46 from which the cutting body 14 is formed. The circumferential direction 34 is chosen so that the base 12 and cutting body 14 tend to lock together when the reamer assembly 10 cuts bone.

It is contemplated to provide reamer assemblies 10, 10' in several embodiments, among them, an embodiment of a fully disposable reamer assembly and a partially disposable reamer assembly. In embodiments where it is contemplated only to dispose of the cutting body 14 and not the base 12, the bayonet slot 28 is formed so as to permit removal of the base 12 from the cutting body 14. This can be accomplished by, for example, forming the spur 40 and or leading edge 52 of the tab 44 so as to permit removal after application of a certain torque, or by providing a tool (not shown) which slides between the spur 40 and tab 44, so as to disable the spur such that it no longer retains the cutting body 14.

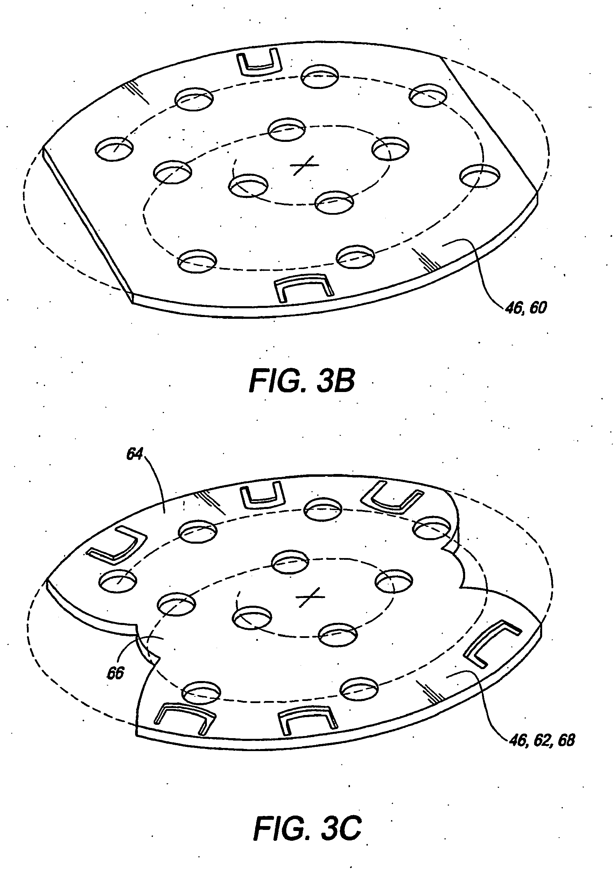

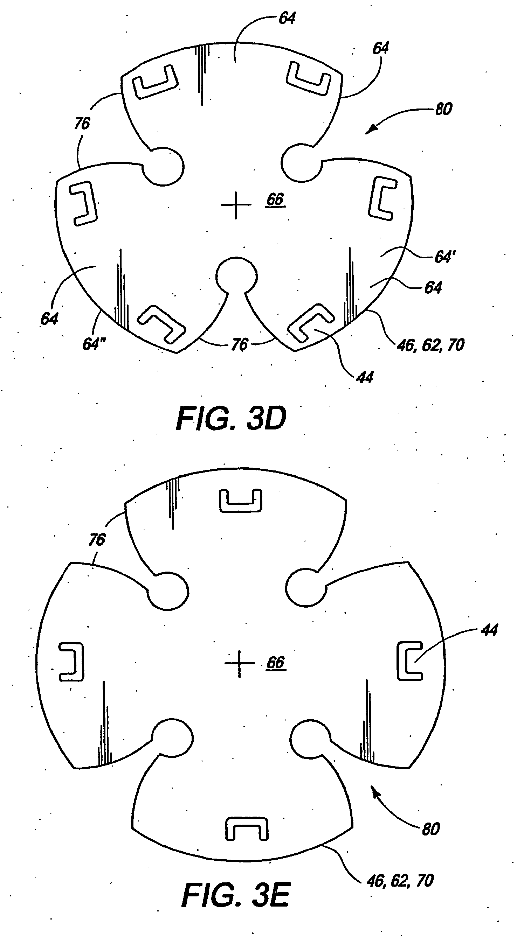

Referring now to FIGs. **3A** to **3E**, in an important aspect of the invention, the blank 46 from which the cutting body 14 is formed is progressively die formed. The blank 46 may be a disk 56 or a partial disk 60, in a manner known for forming a conventional acetabular reamer, or a complex disk 62 having forms having two or more appendages 64 connected at a center 66. Referring in particular to FIG. **3C**, the blank 46 having two appendages 64 connected at a center 66 is a bow tie blank 68. Referring in particular to **FIG. 3D**, the blank 46 having three appendages 64 connected at a center 66 resembles a three-leaf clover and is referred to herein as a three-appendage blank 70. The blank 46 having four appendages connected at a center 66 is a Maltese cross blank 72. The inventors have learned that blanks 46 with appendages 64 may be efficiently die-formed with less overall deformation and thus, such a design is suitable for heavy forming of less ductile materials such as 420 stainless steel, which are usually associated with cutting materials. Thus the die costs less and/or has a longer useful life, thereby reducing the cost of each reamer assembly 10, 10'.

Referring again to FIG. **3B**, the partial disk 60, when fully formed, attaches to a partially circular base 12' so as to minimize the insertion profile of the reamer during minimally invasive surgery ("MIS"). The bow tie blank 68 may be used in this MIS application as well.

Referring now to FIGs. **3D** and **3E**, the three-appendage blank 70 and the Maltese cross blank 72 are suitable for die-forming into a standard hemispherical form 74 as the form can be selected so as to, after die forming, completely occupy the hemispherical shape, wherein adjacent edges 76 butt up one against the other so as to close the gap 80 between such adjacent appendages 64' and 64". Alternatively, a considerable gap 80' (FIG. 1A) may be left.

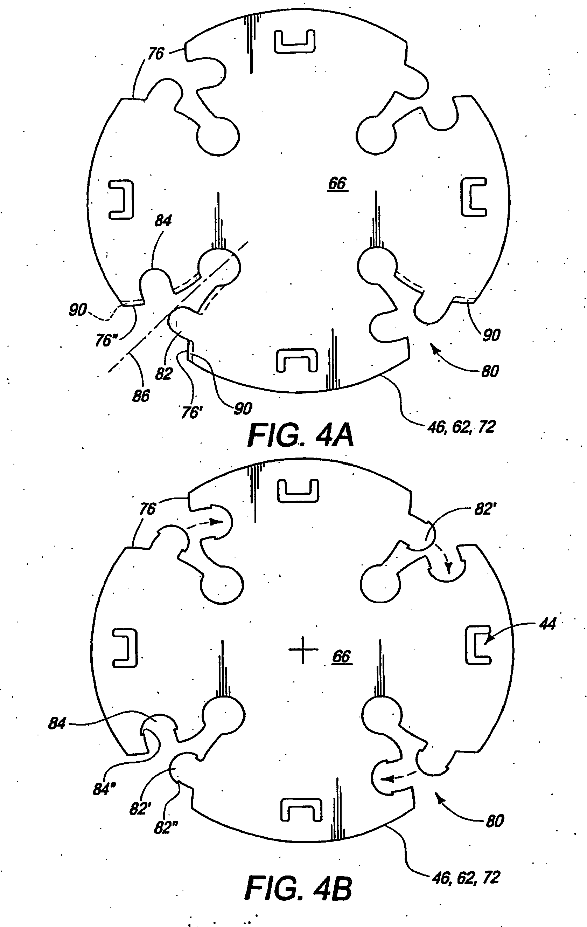

Referring now to FIGs. **4A** and **4B**, optionally, adjacent edges 76' and 76" include male and female interlocks 82 and 84, respectively, which interlock upon completion of the die-forming operation, to help rigidize the cutting form 14. In the embodiment shown, the interlocks help rigidize the cutting form 14 along a line of longitude 86. Alternatively, the interlocks 82' and 84' include spurs 82" which interlock with catches 84" in order to rigidize the cutting form 14 latitudinally as well.

In order to minimize chatter when the reamer assembly 10 is in operation cutting bone, it may be necessary to specially form one or the other of the adjacent edges 76', 76" (leading or trailing) so that it is radiused, or has a slightly curved lip 90 which is curved upwardly or downwardly, with the choice made based on experimental results. At present, the trailing edge (i.e., the edge of an appendage 64 which does not directly faces bone as it is being cut) being curved up slightly (perhaps over a latitudinal length of about 1,27 mm (50 thousandths of an inch)) tends to ensure that bone does not catch on the adjacent leading edge. However, having the adjacent leading edge (i.e., the edge of the appendage 64 that directly faces bone as it is being cut) curved down slightly helps avoid chatter as well.

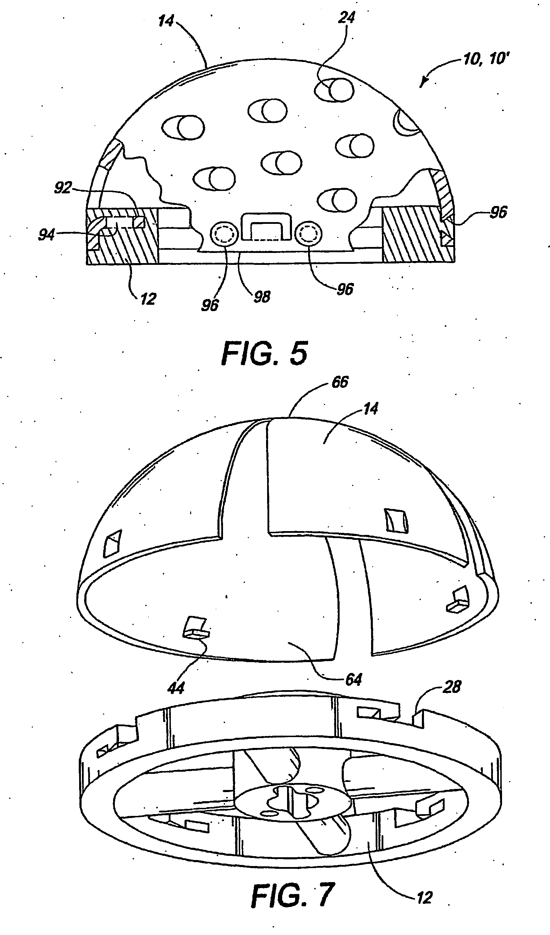

Referring now to FIG. 5, in an alternate embodiment, a reamer assembly 10, 10' may be made in which the interlock between the base 12 and cutting body 14 is accomplished via an insert-molding in which, for example, a large tab 92 having a hole 94 therein, is formed inwardly, about which the base 12 is molded. In addition, or alternatively, holes 96 adjacent the equatorial edge 98, are countersunk from the outside, provide the ability of the molded-over base 12 to fill these countersinks which tend to better hold the assembly together.

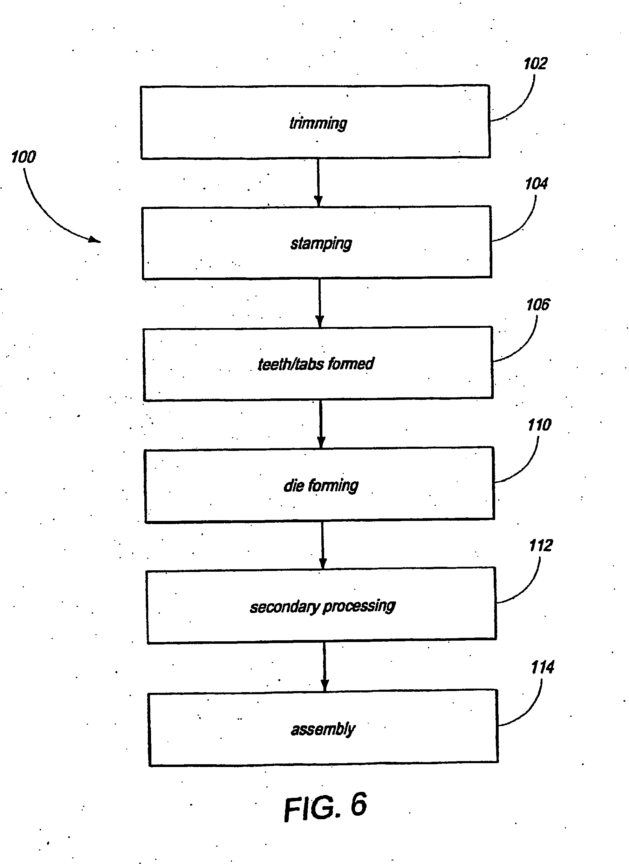

Referring now to FIG. 6, in another embodiment, a method 100 of manufacturing the reamer assembly 10 is provided. The method 100 includes the following steps: In a first step 102, flat stock 46 is trimmed and prepared for die cutting. In a second step 104, the flat stock 46 is die cut to form the appendages 64 connected at a center 66 and, optionally, the perforations 20 are formed at cutting sites 22. Such cutting sites 22 may, at this time, optionally be formed into cutting teeth 24 (or during the die forming step 110 or in subsequent processing steps) In a third step 106, teeth 24 are formed in the die cut flat stock 46, thereby greatly reducing the complexity of forming such teeth, as forming on a pre-formed, flat blank 46 is considerably simpler than forming such teeth on a hemisphere, which would be the case after forming of the body 14. While still in the flat state, the tabs 44 are formed as well. In a fourth step 110, the die cut flat stock 46 is formed into a hemispherical shape as shown in FIG. **1**. In a fifth step 112, secondary processing is performed, such as sharpening the teeth 24 and deburring the body 14. The body 14 is then ready to be affixed via its tabs 44 to the base 12.

In the embodiment (see FIGs. **4A** and **4B**), having interlocks 82' and 84', the male interlock 82' is folded into the recess interlock 84', in a separate step which is taken after die-forming the basic overall convex cutting form 14. This is accomplished by folding the male interlock 82' upward during the stamping step 104, or as a subsequent step, and then receiving these interlocks 82' in reliefs (not shown) in the dies (not shown) immediately under the male interlock 82, so that they are not folded into locking engagement with their corresponding recess interlocks 84' until after the final form is die-formed and the adjacent edges 76' are adjacent one another and the interlocks 82' and 84' properly aligned with one another to facilitate the final step of folding the interlocks 82' into the recess interlocks 84'.

Referring now to FIG. 7, in a final step 114, the cutting body 14 is joined to the base 12, in a mechanical engagement using tabs 44 and bayonet slots 28, or alternatively, as best shown in FIG. 5, by placing the cutting body 14 in a fixture (not shown) and overmolding the base 12 adjacent the equatorial edge 18 of the body.

In an advantage, the invention offers embodiments 46, 50 that permit the disposal of the entire assembly 10 or of the cutting body 14 only.

In an advantage, the appendages 64 assure improved die forming of the body 14 into a final desired shape while at the same time minimizing stress-induced deformation in the body. In other words, the use of appendages 64 assures improved die forming of the body 14 into a final desired shape while at the same time minimizing stress-induced deformation in the body.

In another advantage, the complex teeth 24 can be formed when the body 14 is flat, thereby significantly reducing set up and tool and die costs, while improving accuracy and repeatability.

In another advantage, the complex disks 62 of the invention allow die-forming harder, more durable materials with less warpage than by die-forming a simple disk 56 or partial disk 60.

Multiple variations and modifications are possible in the embodiments of the invention described here. Although certain illustrative embodiments of the invention have been shown and described here, a wide range of modifications, changes, and substitutions is contemplated in the foregoing disclosure. While the above description contains many specifics, these should not be construed as limitations on the scope of the invention, but rather as exemplifications of one or another preferred embodiment thereof. In some instances, some features of the present invention may be employed without a corresponding use of the other features. Accordingly, it is appropriate that the foregoing description be construed broadly and understood as being given by way of illustration and example only, the scope of the invention being limited only by the appended claims.

## Claims

1. An at least partially disposable reamer assembly (10), the reamer assembly including:
a) a rigid, at least partially circular base (12) having an interface for mating with a holder; and
b) a press-formed, thin walled, perforated and toothed cutting body (14) having interlocking interfaces for locking with the base (12), wherein the base (12) and body (14) connect via a mechanical connection, **characterized in that**
the body (14) connects to the base (12) via tabs (44) in the body (14) that enter bayonet slots (28) in the base (12), wherein the tabs (44) extend inwardly into the cutting body (14).

2. The reamer assembly of claim 1, wherein the base (12) is molded of a biocompatible plastic.

3. The reamer assembly of claim 2, wherein the base (12) is characterized as having been insert molded in the cutting body (14) to form a fully disposable reamer assembly (10).

4. The reamer assembly of claim 2, wherein the base (12) is characterized as having been over-molded on to the cutting body (14), thus forming a completely disposable reamer assembly.

5. The reamer assembly of claim 1, wherein the locking interfaces (16) interlock further when the reamer assembly is rotated in a cutting direction.

6. The reamer assembly of claim 1, wherein the first locking interface includes a spur (40) which tends to lock the second locking interface in the first locking interface.

7. The reamer assembly of claim 1, wherein the locking interfaces unlock when the reamer assembly is rotated opposite a cutting direction, thereby permitting disassembly of the assembly.

8. The reamer assembly of claim 1, wherein the cutting body (14) is characterized as having been progressive die-formed.

9. The reamer assembly of claim 1, wherein the cutting body (14) is characterized as having been die-formed of a stamped, flat metal blank in the form resembling a Maltese cross.

10. The reamer assembly of claim 1, wherein the cutting body (14) is characterized as having been die-formed of a stamped, flat metal blank having three extending appendages connected at a center.

11. The reamer assembly of claim 1, wherein the cutting body (14) is characterized as having been die-formed of a stamped, flat metal blank having two extending appendages connected at a center.

12. The reamer assembly of claim 1, wherein the cutting body (14) is characterized as having been die-formed of a stamped, flat metal blank.

## Patentansprüche

1. Fräseanordnung (10), die zumindest teilweise wegwerfbar ist, wobei die Fräseanordnung aufweist:
a) eine starre, zumindest teilweise kreisförmige Basis (12) mit einer Schnittstelle zum Verbinden mit einem Halter; und
b) einen press-geformten, dünnwandigen, perforierten und gezahnten Schneidekörper (14) mit verriegelbaren Schnittstellen zum Verriegeln mit der Basis (12), wobei sich die Basis (12) und der Körper (14) über eine mechanische Verbindung verbinden, **dadurch gekennzeichnet, dass**
sich der Körper (14) mit der Basis (12) über Vorsprünge (44) in dem Körper (14) verbindet, die in Bajonettöffnungen (28) in der Basis (12) eindringen, wobei sich die Vorsprünge (44) einwärts in den Schneidekörper (14) erstrecken.

2. Fräseanordnung nach Anspruch 1, wobei die Basis (12) aus einem biokompatiblen Plastik geformt ist.

3. Fräseanordnung nach Anspruch 2, wobei die Basis (12) gekennzeichnet ist, als wenn sie durch Inserttechnik in den Schneidekörper (14) geformt ist, um eine vollkommen wegwerfbare Fräseanordnung (10) zu formen.

4. Fräseanordnung nach Anspruch 2, wobei die Basis (12) gekennzeichnet ist, als wenn sie durch Überformen auf den Schneidekörper (14) geformt ist, wobei demnach eine ganz wegwerfbare Fräseanordnung geformt wird.

5. Fräseanordnung nach Anspruch 1, wobei sich die verriegelbaren Schnittstellen (16) weiter verriegeln, wenn die Fräseanordnung in eine Schneiderichtung gedreht wird.

6. Fräseanordnung nach Anspruch 1, wobei die erste verriegelbare Schnittstelle einen Vorsprung (40) aufweist, der dazu tendiert die zweite verriegelbare Schnittstelle in der ersten verriegelbaren Schnittstelle zu verriegeln.

7. Fräseanordnung nach Anspruch 1, wobei sich die verriegelbaren Schnittstellen entriegeln, wenn die Fräseanordnung entgegengesetzt einer Schneiderichtung gedreht wird, wobei dadurch die Demontage von der Anordnung ermöglicht wird.

8. Fräseanordnung nach Anspruch 1, wobei der Schneidekörper (14) gekennzeichnet ist, als wenn er progressiv press-geformt ist.

9. Fräseanordnung nach Anspruch 1, wobei der Schneidekörper (14) gekennzeichnet ist, als wenn er aus einem gestempelten, flachen Metallstück, in der Form die einem Malteserkreuz gleicht, press-geformt ist.

10. Fräseanordnung nach Anspruch 1, wobei der Schneidekörper (14) gekennzeichnet ist, als wenn er aus einem gestempelten, flachen Metallstück press-geformt ist, das drei sich erstreckende Abschnitte aufweist, die in einem Zentrum verbunden sind.

11. Fräseanordnung nach Anspruch 1, wobei der Schneidekörper (14) gekennzeichnet ist, als wenn er aus einem gestempelten, flachen Metallstück press-geformt ist, das zwei sich erstreckende Abschnitte aufweist, die in einem Zentrum verbunden sind.

12. Fräseanordnung nach Anspruch 1, wobei der Schneidekörper (14) gekennzeichnet ist, als wenn er aus einem gestempelten, flachen Metallstück press-geformt ist.

## Revendications

1. Ensemble alésoir au moins partiellement jetable (10), l'ensemble alésoir comprenant :
a) une base rigide au moins partiellement circulaire (12) ayant une interface d'accouplement avec un support ; et
b) un corps de coupe formé sous pression, à parois fines, perforé et denté (14) ayant des interfaces d'enclenchement pour un enclenchement avec la base (12), dans lequel la base (12) et le corps (14) sont reliés via une liaison mécanique, **caractérisé en ce que**
le corps (14) est relié à la base (12) via des pattes (44) dans le corps (14) qui entrent dans des fentes à baïonnette (28) dans la base (12), dans lequel les pattes (44) s'étendent vers l'intérieur dans le corps de coupe (14).

2. Ensemble alésoir selon la revendication 1, dans lequel la base (12) est réalisée par moulage d'un plastic biocompatible.

3. Ensemble alésoir selon la revendication 2, dans lequel la base (12) est **caractérisée en ce qu'**elle a été insérée moulée dans le corps de coupe (14) pour former un ensemble alésoir entièrement jetable (10).

4. Ensemble alésoir selon la revendication 2, dans lequel la base (12) est **caractérisée en ce qu'**elle a été surmoulée sur le corps de coupe (14), formant ainsi un ensemble alésoir entièrement jetable.

5. Ensemble alésoir selon la revendication 1, dans lequel les interfaces d'enclenchement (16) s'enclenchent en outre lorsque l'ensemble alésoir effectue une rotation dans une direction de coupe.

6. Ensemble alésoir selon la revendication 1, dans lequel la première interface d'enclenchement comprend un ergot (40) qui tend à enclencher la deuxième interface d'enclenchement dans la première interface d'enclenchement.

7. Ensemble alésoir selon la revendication 1, dans lequel les interfaces d'enclenchement se désenclenchent lorsque l'ensemble alésoir effectue une rotation à l'opposé d'une direction de coupe, permettant ainsi le désassemblage de l'ensemble.

8. Ensemble alésoir selon la revendication 1, dans lequel le corps de coupe (14) est **caractérisé en ce qu'**il a été formé par matriçage progressif.

9. Ensemble alésoir selon la revendication 1, dans lequel le corps de coupe (14) est **caractérisé en ce qu'**il a été formé par matriçage d'une ébauche en métal plate emboutie sous la forme d'une croix de Malte.

10. Ensemble alésoir selon la revendication 1, dans lequel le corps de coupe (14) est **caractérisé en ce qu'**il a été formé par matriçage d'une ébauche en métal plate emboutie ayant trois appendices se prolongeant reliés au niveau d'un centre.

11. Ensemble alésoir selon la revendication 1, dans lequel le corps de coupe (14) est **caractérisé en ce qu'**il a été formé par matriçage d'une ébauche en métal plate emboutie ayant deux appendices se prolongeant reliés au niveau d'un centre.

12. Ensemble alésoir selon la revendication 1, dans lequel le corps de coupe (14) est **caractérisé en ce qu'**il a été formé par matriçage d'une ébauche en métal plate emboutie.
